# EUROPEAN PATENT SPECIFICATION

(11) **EP 1 336 394 B1**
(45) Date of publication and mention of the grant of the patent: **27.09.2006**
(21) Application number: 03003329.4
(22) Date of filing: 13.02.2003
(51) Int. Cl.: A61F 2/34

(54) **Acetabular prosthesis of the hip**
Gelenkpfannenprothese der Hüfte
Prothèse acétabulaire de la hanche

(30) Priority: 14.02.2002 IT UD20020037
(43) Date of publication of application: 20.08.2003
(73) Proprietor: LIMA Lto SpA, 33030 Villanova di San Daniele del Friuli (UD) (IT)
(72) Inventor: Dalla Pria, Paolo, 33100 Udine (IT)
(74) Representative: Petraz, Gilberto Luigi

(56) References cited:
- EP-A- 0 308 297
- EP-A- 0 773 007
- EP-A- 1 072 235
- EP-A- 1 082 949
- WO-A-85/00284
- DE-A- 19 701 778
- DE-A- 19 746 997
- DE-C- 19 620 750
- FR-A- 2 700 947
- FR-A- 2 753 081
- FR-A- 2 786 390

## Description

### FIELD OF THE INVENTION

The present invention concerns an acetabular prosthesis comprising as its base elements an outer shell able to be inserted into a natural acetabular seating of a hip and a modular insert able to house in its turn a femoral prosthesis.

The modular insert comprises alignment means by means of which it is able to be coupled univocally to the outer shell according to at least two pre-determined positions diversely oriented angularly with respect to at least a reference plane. These different positions can be obtained by means of a simple rotation of the modular insert with respect to the outer shell.

Moreover, the modular insert is interchangeable with others of the same type which however allow to obtain orientation with respect to different planes and/or respective adaptations to the left or right side of the hip.

### BACKGROUND OF THE INVENTION

In the field of hip prostheses, the problems concerning the correct orientation which the artificial acetabular cup must assume in order to guarantee a correct efficiency of the coxo-femoral prosthesis are well-known.

It is in fact known that, due to degenerative phenomena, traumas or in the case of a revision of previous implants, the bone wall where the acetabular cup is inserted may be missing, recessive or partly damaged. In these cases, the angular orientation assumed by the cup in order to guarantee in any case an adequate anchorage to the bone, often determines a reduced covering surface for the femoral head, and this entails the risk of accidental slippages, or dislocations, of the head itself, or even anomalous wear or damage to the components.

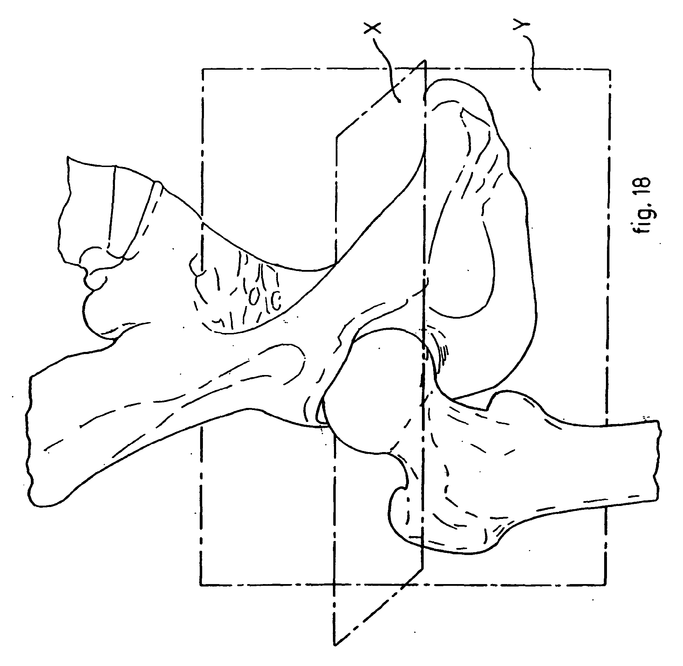

In order to obtain an efficient and long-lasting functioning of the components of the prosthesis it is therefore often necessary, according to the conditions of the natural acetabular seating, to be able to modify the angle of the articular insert, with reference to the articulation of the hip, both with respect to a frontal or coronal plane Y, substantially vertical, and also with respect to a transverse plane X, substantially horizontal. Fig. 18 shows a schematic representation of these two planes of reference.

The lateral orientation of the acetabular cup with respect to the frontal plane is normally defined angle of cover, while the orientation with respect to the transverse plane, which corresponds to a more or less "forward" positioning of the cup, is normally defined angle of anteversion.

To be more exact, the angle of cover is normally defined as the angle between the projection onto the frontal plane of the equator of the acetabular cup and a horizontal line located on the same frontal plane; the angle of anteversion, instead, is defined as the angle between the projection onto the transverse plane of the equator of the acetabular cup and a horizontal line located on the same transverse plane.

To solve the disadvantages described above, there have been proposals for acetabular cups comprising an outer shell and an intermediate insert, wherein the positioning axis of the insert is angled for a desired value with respect to the axis of symmetry of the outer shell.

For example, the present Applicant has embodied in document EP-A-1 082 949, on which claim 1 is characterized, an acetabular prosthesis wherein on the wall of the intermediate insert and on that of the outer shell there are a plurality of holes into which screws can be inserted for reciprocal attachment.

The intermediate insert can be oriented in various angular positions provided that at least one hole of the outer cup is aligned with a hole of the intermediate insert.

This acetabular prosthesis of a conventional type has the disadvantage, however, that it is not very easy to use because the surgeon, during the operation, does not have a precise spatial reference in order to be able to determine the optimum angular position and to choose the correct orientation of the intermediate insert. Moreover, the surgeon is often obliged to make several attempts to find the alignment between the holes and to insert the relative screw.

It must also be considered that, during the operating step, the space available to make all the operations is extremely limited, and this entails another obstacle for precision of positioning and ease of movement.

WO 85/00284 A discloses a hip prosthesis comprising an outer shell and an inner cup having a spherical inner seating wherein articulates the head of a femoral prosthesis. The inner cup may be mounted in the outer shell according to various angular interadjustements of the respective contact surfaces. In order to determine the direction of the articulation independently of the insertion direction of the outer shell into the bony acetabulum, the direction of the articulation surface of the inner cup differs from the direction of the shell middle axis.

The present Applicant has devised and embodied this invention to overcome these shortcomings of the state of the art and to obtain further advantages.

### SUMMARY OF THE INVENTION

The present invention is set forth and characterized in the main claim, while the dependent claims describe other characteristics of the invention.

The purpose of the present invention is to achieve an acetabular prosthesis with which it is possible to obtain a plurality of pre-determined positions and angular orientations of an insert with respect to an outer shell, ensuring, substantially in all cases which might be found, a sufficient protection and covering of the head of a femoral prosthesis.

Another purpose of the invention is to obtain an acetabular prosthesis which can be inserted into a pre-determined angular position both in a right acetabular seating and also in a left acetabular seating.

Another purpose is to facilitate the task of the surgeon in operations to position and attach the prosthesis since, once the conditions of the bone seating have been recognized in the preparatory stage, he immediately knows which components to use and how to position them.

In accordance with these purposes, an acetabular prosthesis according to the present invention comprises an outer shell, able to be inserted and attached, according to a pre-determined direction of implantation, in a relative bone seating of a hip. The outer shell has an inner cavity into which an insert is able to be inserted, whose outer conformation is suitable to couple with the inner cavity of the shell according to a defined coupling axis.

The insert has in turn an inner cavity into which a possible articular element can be inserted which functions as a positioning and articulation seating for the head of the femoral prosthesis.

According to a first characteristic of the present invention, the insert comprises alignment means by means of which it is able to be coupled univocally to the outer shell according to at least two pre-determined positions, diversely oriented at an angle with respect to at least one plane of reference.

According to another characteristic, the insert has an inner positioning seating, asymmetrical and angled by a desired angle with respect to the direction of implantation of the outer shell.

Thanks to these characteristics, the positioning of the insert in one or the other of the pre-determined positions which it can assume with respect to the outer shell, which can be obtained by means of a rotation through 180° in the case of two selectable positions, determines an automatic and pre-determined variation in the orientation which the femoral prosthesis will assume with respect to the direction of implantation of the outer shell.

According to the invention, the acetabular prosthesis comprises at least a first module able to define, with reference to the frontal or coronal plane, two respective and distinct angles of cover which can be obtained by positioning a first type of insert in its two positions selectable with respect to the axis of implantation.

The acetabular prosthesis according to the invention also comprises a second module, which uses a second type of insert whose inner configuration is designed so as to allow to obtain a defined angle of anteversion with respect to the transverse plane, with an angle of cover which varies according to its positioning on one side, left or right, of the hip, and a third module, specular to the second module, which uses a third type of insert, which allows to obtain the same angle of anteversion but specular values of the angle of cover on the opposite side of the hip.

According to another characteristic of the present invention, the coupling between the outer shell and the insert is of the conical type, wherein the axis of the coupling cone is angled by a certain value with respect to the axis of symmetry of the outer shell.

Thanks to this conical coupling, when it is inserted into the inner cavity of the outer shell, the insert is already automatically oriented with a certain angle with respect to the axis of symmetry of the outer shell.

By suitably designing the inner cavities of the insert it is possible to obtain, in this way, a first condition wherein the angle of cover, with respect to the frontal plane, is zero since the angular orientations, respectively between inside and outside the shell and between inside and outside the insert, compensate for each other and cancel each other; and a second condition,' by rotating the insert through 180°, wherein said orientations are added together, in order to obtain the desired value of the angle of cover equal to the sum of the angular orientations defined in the design phase.

### BRIEF DESCRIPTION OF THE DRAWINGS

These and other characteristics of the present invention will become apparent from the following description of a preferential form of embodiment, given as a non-restrictive example with reference to the attached drawings wherein:
- fig. 1 is a front view, partly in section, of the acetabular prosthesis according to the invention during the coupling step;
- fig. 2 is a front view of the prosthesis in fig. 1 in a first coupling position between the outer shell and insert;
- fig. 3 is a side view of fig. 2;
- fig. 4 is a front section of the prosthesis in fig. 2;
- fig. 5 is a front view of the prosthesis in fig. 1 in a second coupling position rotated through 180°;
- fig. 6 is a side view of fig. 5;
- fig. 7 is a front section of the prosthesis in fig. 5;
- fig. 8 is a front view of an acetabular prosthesis according to the invention using a second type of insert, in a position of coupling with the outer shell;
- fig. 9 is a side view of fig. 8;
- fig. 10 shows the prosthesis with the insert of fig. 9 rotated through 180°;
- fig. 11 is a side view of fig. 10;
- fig. 12 is a front view of an acetabular prosthesis according to the invention using a third type of insert, in a coupling position;
- fig. 13 is a side view of fig. 12;
- fig. 14 shows the prosthesis with the insert of fig. 12 rotated through 180°;
- fig. 15 is a side view of fig. 14;
- figs. 16 and 17 show respectively an outer shell and an insert according to a variant of the invention;
- fig. 18 is a schematic representation of the planes of reference of an articulation of the hip.

### DETAILED DESCRIPTION OF A PREFERENTIAL EMBODIMENT

With reference to the attached figures, an acetabular prosthesis 10 according to the present invention comprises an outer shell 12, with a substantially hemispherical outer shape, which has an inner cavity 14 able to house an insert 16 of a modular type. The modular insert 16 has in turn an inner cavity 16a into which is inserted, due to its having the same shape, an articular element which is not shown here, preferably made of anti-wear material, such as polyethylene, metal or ceramic, able to house the head of a femoral prosthesis.

The outer shell 12 is able to be inserted and attached in an acetabular bone seating of a hip.

As mentioned above, the angular orientation in space of the outer shell 12 and of the modular insert 16 can be defined with respect to two main planes (fig. 18), that is, a frontal or coronal plane Y, substantially vertical, and a transverse plane X, substantially horizontal and orthogonal to the first.

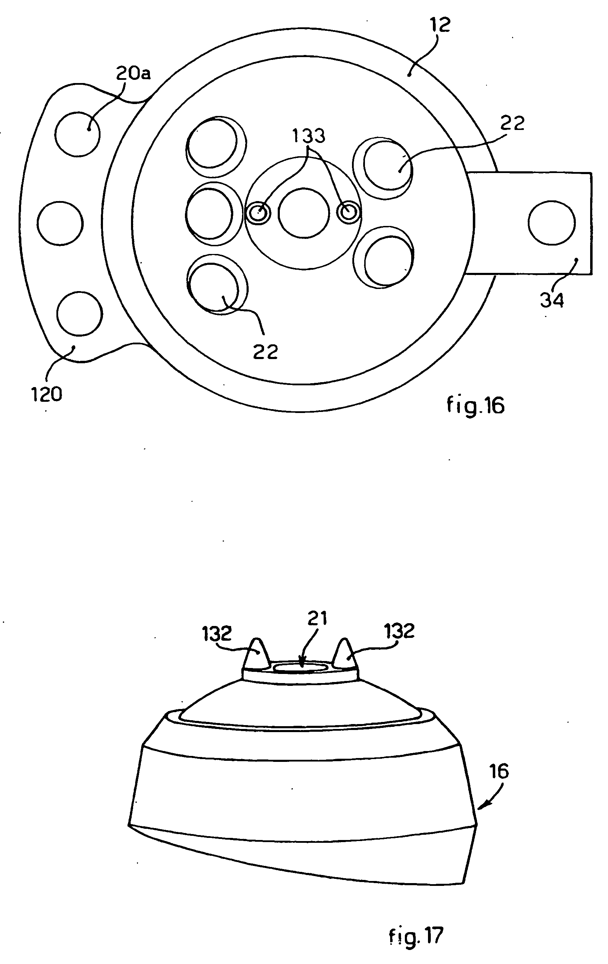

The outer shell 12 is attached to the bone seating and, in order to be attached, it comprises on one side fins 20 or a flange 120 (fig. 16), having a plurality of holes 20a in which to insert anchoring screws or nails onto the bone wall surrounding the acetabular seating. There may also be a hook 34 which facilitates the anchorage under particular conditions of the bone seating.

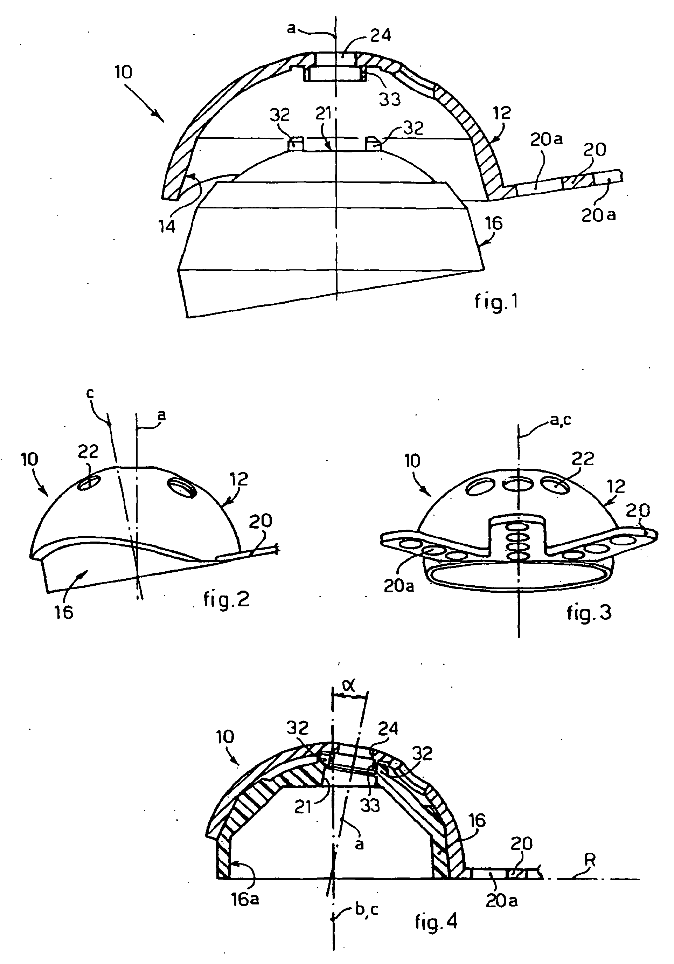

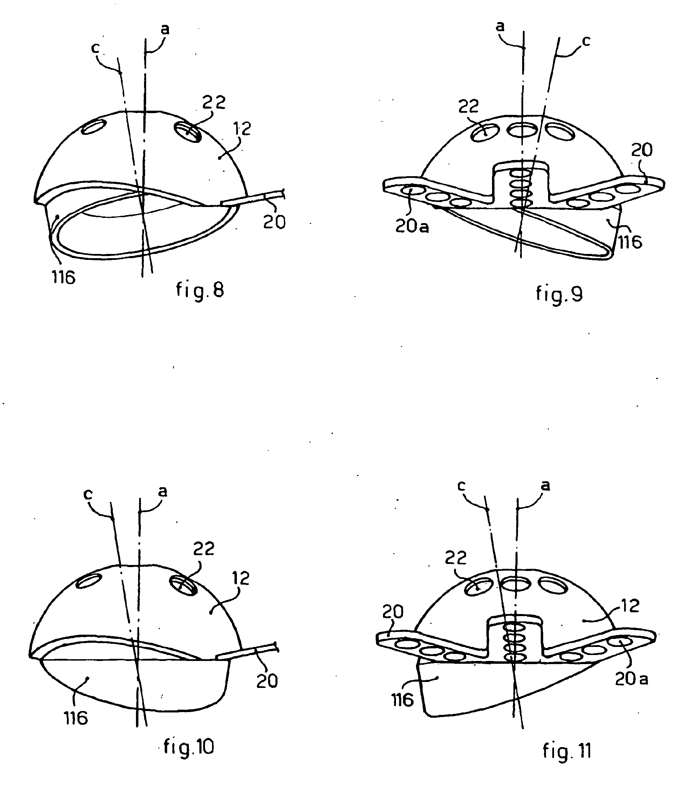

The fins 20 or flange 120 define a reference plane R with respect to which the angle of cover of the acetabular cup is measured (figs. 4 and 7). On the outside the shell 12 also has other holes 22, into which further attachment means can be inserted.

In the preferential embodiment of the invention, the coupling between the outer shell 12 and the modular insert 16 is of the conical type, and is achieved by means of alignment means, such as pins 32, able to cooperate with mating retaining means, in this case a key-type seating 33, made on the inner surface of the shell 12 (fig. 1).

It is obvious that other types of mating alignment and retaining means can be provided, such as conical stakes 132 cooperating with holes 133 (as in the solution shown in figs. 16 and 17), pins and fins, or other means having the same function.

The pins 32 or stakes 132 allow to position the modular insert 16 with respect to the outer shell 12 only according to two pre-determined positions, rotated through 180° with respect to each other. In these positions, in the solution shown, a through hole 21 present on the surface of the insert 16 is aligned with a threaded through hole 24 present on the surface of the shell 12, in order to insert an attachment screw which reciprocally clamps the two elements.

It may not be necessary, however, to use an attachment screw.

In this case, the axis "a" of the coupling cone between the shell 12 and the insert 16 is inclined by a certain angle "α", for example about 10°, with respect to the orthogonal "b" to the plane of reference R defined by the attachment fins 20 (fig. 4). It comes within the field of the invention that said angle α can assume a variable design value, for example between about 5° and about 45°.

In turn, the inner cavity 16a of the modular insert 16 is angularly offset with respect to the axis "a" of the coupling cone by an angle which, as an absolute value, is advantageously equal to said angle "α".

With this design configuration, an articular insert, not shown here, which is positioned due to its having the same shape in said cavity 16a, is angularly oriented with respect to the reference plane R by an angle whose value depends on the position which the modular insert 16 assumes with respect to the shell 12.

With reference to figs. 1 to 7, and in particular to figs. 4 and 7, it can be seen how the modular insert 16 can be positioned in a first position (fig. 4) wherein the axis of symmetry "c" of its inner cavity 16a substantially coincides with the orthogonal "b" to the reference plane R, and a second position (fig. 7), rotated through 180° with respect to the first, wherein the axis of symmetry "c" is angled by an angle equal to "2α" with respect to said orthogonal "b".

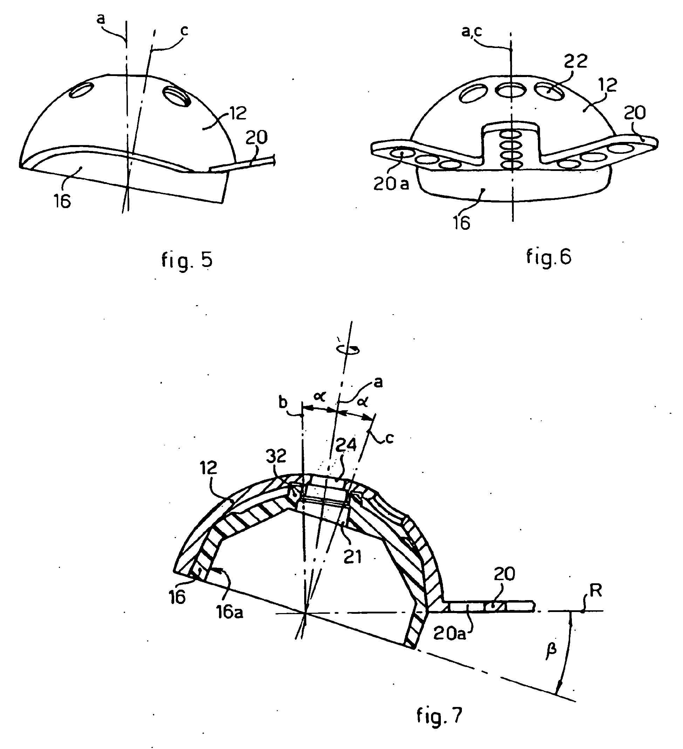

In the first position (figs. 2-4), the two angular shiftings, respectively between the outside and inside of the shell 12 and the outside and inside of the insert 16, compensate for each other, and therefore the resultant angle of cover is zero. In the second position (figs. 5-7), the two angular shiftings are added to each other and therefore we obtain an overall angle of cover "β" = "2α", in this case equal to 20°.

Figs. 2 and 3 show the condition of absence of cover, while figs. 5 and 6 show the same insert 16 rotated through 180° in order to obtain the desired cover condition.

Since the insert 16 has a symmetrical conformation with respect to the frontal plane Y, it defines a zero angle of anteversion, and therefore can be used indifferently on both left and right hips. Obviously, a particular orientation of the insert 16 will produce a zero angle of cover "β" for the right hip and 20° for the left hip, while the orientation rotated through 180° will produce an angle of cover of 20° for the right hip and zero for the left hip.

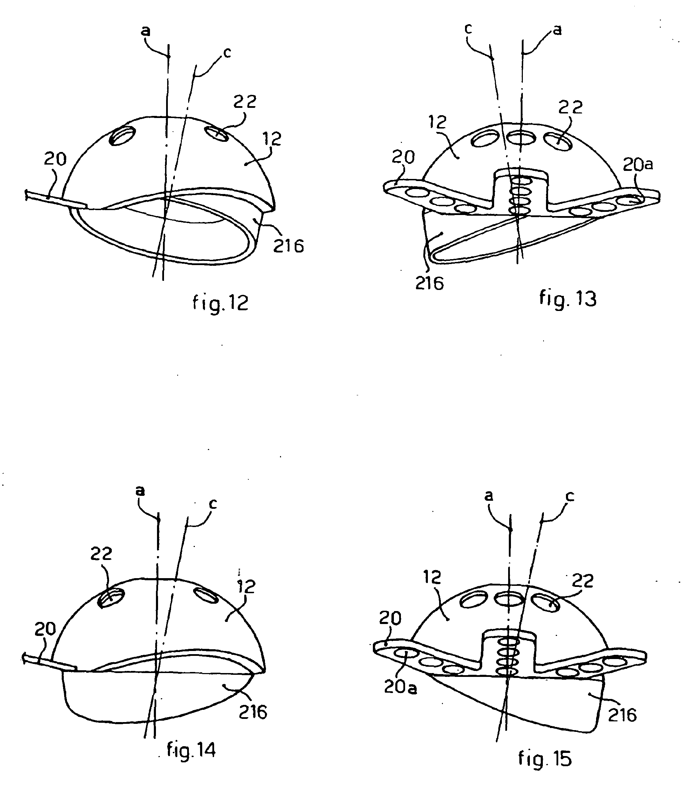

Instead of the insert 16, having a zero angle of anteversion (that is, a zero forward orientation with respect to the transverse plane X), when it is necessary to obtain an angle of anteversion too, two other types of insert can be used, respectively 116 (figs. 8-11) and 216 (figs. 12-15), whose conformation is asymmetrical with respect to the frontal plane Y.

The two inserts 116 and 216 allow to obtain a defined angle of anteversion during the design stage, for example of about 15°, while still maintaining the same covering properties on the frontal plane of the modular insert 16 of the first type.

With the same principle described above, by rotating the insert 116 or 216 through 180° with respect to said coupling axis "a", it is possible to vary the angle of cover, for example taking it from 0° to 20°, as in the previous case, in any case keeping the angle of anteversion equal to 15°.

However, the use of two types of different inserts, respectively 116 and 216, is necessary to obtain all the options of cover and anteversion both for the right and for the left hips.

In fact, as can be seen in figs. 8 to 11, a first insert 116 allows to obtain, in a first coupling position with the outer shell 12, an angle of anteversion condition equal to 15° and a zero angle of cover (figs. 8 and 9) with respect to the plane of reference R defined by the fins 20.

In the second position (figs. 10 and 11), rotated through 180° with respect to the first, the same insert 116 allows to obtain, but for the opposite hip, an angle of anteversion condition again equal to 15°, but with an angle of cover which is not zero, for example equal to 20° in the design situation as described above.

This is due to the fact that the rotation through 180° of the insert 116 causes the asymmetry that determines the presence of an angle of anteversion which is not zero is now referred to the frontal plane which passes through the opposite hip.

The insert 216 is specular to the insert 116 and allows to obtain specular conditions, that is, zero cover and anteversion, in this case, equal to 15° for a first side of the hip (figs. 12 and 13) opposite that of figs. 8 and 9, and 20° cover and 15° anteversion for the opposite hip, by rotating the insert itself through 180° (figs. 14 and 15).

It is clear that modifications or additions can be made to the acetabular prosthesis 10 as described heretofore, without departing from the field and scope of the present invention. It is also clear that, although the description refers to a specific example, the person of skill in the art shall be able to achieve other equivalent forms of acetabular prosthesis 10, all of which shall come within the field and scope of the present invention.

## Claims

1. Acetabular prosthesis of a hip comprising at least an outer shell (12), able to be inserted and fixed in a relative bone seating, and an insert able to be inserted into said outer shell (12) and to house an articular element able in turn to house the head of a femoral prosthesis, **characterized in that** said insert (16, 116, 216) comprises alignment means (32, 132) for univocally coupling said insert (16, 116, 216) to said outer shell (12) according to at least two alternate pre-determined positions diversely oriented angularly with respect to at least one plane of reference, wherein said at least two alternate pre-determined positions comprise a first position and a second position, in which second position said insert (16, 116, 216) is rotated relative to said first position with respect to an axis ("a") of coupling with said outer shell (12), each of said predetermined positions defining a respective and different value of an angle of cover of the insert (16, 116, 216) with respect to said plane of reference.

2. Acetabular prosthesis as in claim 1, **characterised in that** in said second position said insert (16, 116, 216) is rotated relative to the first position through 180° with respect to an axis ("a") of coupling with said outer shell (12).

3. Acetabular prosthesis as in claim 1 or 2, **characterized in that** said insert (16, 116, 216) has an inner positioning seating (16a) angled at a predetermined angle (α) with respect to the coupling axis ("a") between the insert and outer shell (12).

4. Acetabular prosthesis as in claim 3, comprising means (20, 120) of attachment to the bone seating defining a plane of reference (R) with respect to which an angle of cover of said insert (16, 116, 216) is measured, **characterized in that** said coupling axis ("a") between the outer shell (12) and the insert (16, 116, 216) is inclined by said predetermined angle ("α") with respect to an orthogonal ("b") to said plane of reference (R).

5. Acetabular prosthesis as in any claim hereinbefore, **characterized in that** the coupling between the outer shell (12) and the insert (16, 116, 216) is of the conical type with a coupling axis ("a").

6. Acetabular prosthesis as in claim 4, **characterized in that** said insert is at least of a first type (16) symmetrical on a frontal plane (Y) substantially vertical with respect to the hip, in order to vary the angle of cover with respect to said plane of reference (R) in conditions of angle of anteversion of zero on a transverse plane (X) substantially horizontal with respect to the hip.

7. Acetabular prosthesis as in claim 4, **characterized in that** said insert is respectively of a second (116) and third (216) type asymmetrical with respect to a frontal plane (Y) substantially vertical with respect to the hip in order to vary the angle of cover with respect to said plane of reference (R) in conditions of angle of anteversion not of zero on a transverse plane (X) substantially horizontal with respect to the hip.

8. Acetabular prosthesis as in claim 7, **characterized in that** said insert (116) of the second type has a specular conformation with respect to said insert (216) of the third type in order to define specular cover conditions between the right hip and the left hip in conditions of angle of anteversion not of zero.

9. Acetabular prosthesis as in any claim hereinbefore, **characterized in that** the angle ("α") formed between the coupling axis ("a") between said outer shell (12) and said insert (16, 116, 216), and said orthogonal ("b") to the plane of reference (R) is equal, as an absolute value, to the angle between the axis of the inner positioning seating (16a) of said insert and said coupling axis ("a"), so that there exists at least a position of said insert (16, 116, 216) which defines an angle of cover of zero with respect to said plane of reference (R).

10. Acetabular prosthesis as in any claim hereinbefore, **characterized in that** said alignment means comprise a pair of protrusions or pins (32, 132) made on the outer surface of said insert (16, 116, 216) and able to cooperate with mating retaining means (33, 133) present on the inner surface of said outer shell (12) in order to define said pre-determined positions of said insert (16, 116, 216) with respect to said shell (12).

11. Acetabular prosthesis as in any claim hereinbefore, **characterized in that** said pre-determined angle (α) has a value of between 5 and 45°.

12. Acetabular prosthesis as in any claim hereinbefore, **characterized in that** said inserts (116, 216) of the second and third type are able to define an angle of anteversion of about 15°.

## Patentansprüche

1. Hüftpfannenprothese mit zumindest einer äußeren Schale (12), die in einer zugehörigen Knochenauflagefläche eingesetzt und befestigt werden kann, und einem Einsatz, der in die äußere Schale (12) eingesetzt werden und ein Gelenkelement aufnehmen kann, das wiederum den Kopf einer Oberschenkelprothese aufnehmen kann, **dadurch gekennzeichnet, dass** der Einsatz (16, 116, 216) ein Ausrichtungsmittel (32, 132) zum eindeutigen Koppeln des Einsatzes (16, 116, 216) an der äußeren Schale (12) zumindest gemäß zwei abwechselnden vorbestimmten Positionen umfasst, die vom Winkel her mit Bezug auf zumindest eine Bezugsebene unterschiedlich ausgerichtet sind, wobei zumindest zwei abwechselnde vorbestimmte Positionen eine erste Position und eine zweite Position umfassen, wobei in der zweiten Position der Einsatz (16, 116, 216) relativ zu der ersten Position mit Bezug auf eine Kopplungsachse ("a") mit der äußeren Schale (12) gedreht ist, wobei jede der vorbestimmten Positionen einen entsprechenden und unterschiedlichen Wert eines Abdeckwinkels des Einsatzes (16, 116, 216) mit Bezug auf die Bezugsebene definiert.

2. Hüftpfannenprothese nach Anspruch 1, **dadurch gekennzeichnet, dass** in der zweiten Position der Einsatz (16, 116, 216) relativ zu der ersten Position um 180° mit Bezug auf eine Kopplungsachse ("a") mit der äußeren Schale (12) gedreht ist.

3. Hüftpfannenprothese nach Anspruch 1 oder 2, **dadurch gekennzeichnet, dass** der Einsatz (16, 116, 216) eine innere Positionierauflagefläche (16a) hat, die bei einem vorbestimmten Winkel (α) mit Bezug auf die Kopplungsachse ("a") zwischen dem Einsatz und dem äußeren Gehäuse gewinkelt ist.

4. Hüftpfannenprothese nach Anspruch 3 mit einem Mittel (20, 120) zur Befestigung an der Knochenauflagefläche, die eine Bezugsebene (R) definiert, mit Bezug auf die ein Abdeckwinkel des Einsatzes (16, 16, 216) gemessen wird, **dadurch gekennzeichnet, dass** die Kopplungsachse ("a") zwischen der äußeren Schale (12) und dem Einsatz (16, 116, 216) um den vorbestimmten Winkel ("α") mit Bezug auf eine Orthogonale ("b") zu der Bezugsebene (R) geneigt ist.

5. Hüftpfannenprothese nach irgendeinem der vorhergehenden Ansprüche, **dadurch gekennzeichnet, dass** die Kopplung zwischen der äußeren Schale (12) und dem Einsatz (16, 116, 216) vom konischen Typ mit einer Kopplungsachse ("a") ist.

6. Hüftpfannenprothese nach Anspruch 4, **dadurch gekennzeichnet, dass** der Einsatz zumindest von einem ersten Typ (16) ist, der symmetrisch auf einer vorderen Ebene (Y) steht, die im Wesentlichen vertikal mit Bezug auf die Hüfte ist, um den Abdeckwinkel mit Bezug auf die Bezugsebene (R) zu Bedingungen eines Anteversionswinkels von Null zu einer transversalen Ebene (X) zu variieren, die im Wesentlichen horizontal mit Bezug auf die Hüfte ist.

7. Hüftpfannenprothese nach Anspruch 4, **dadurch gekennzeichnet, dass** der Einsatz von einem zweiten (116) bzw. dritten (216) Typ ist, der asymmetrisch mit Bezug auf eine vordere Ebene (Y) ist, die im Wesentlichen vertikal mit Bezug auf die Hüfte ist, um den Abdeckwinkel mit Bezug auf die Bezugsebene (R) unter Bedingungen eines Anteversionswinkels zu variieren, der zu einer transversale Ebene (X) nicht Null ist, die im Wesentlichen horizontal mit Bezug auf die Hüfte ist.

8. Hüftpfannenprothese nach Anspruch 7, **dadurch gekennzeichnet, dass** der Einsatz (16) des zweiten Typs eine gespiegelte Formgebung mit Bezug auf den Einsatz (216) des dritten Typs hat, um gespiegelte Abdeckbedingungen zwischen der rechten Hüfte und der linken Hüfte unter Bedingungen des Anteversionswinkels zu definieren, der ungleich Null ist.

9. Hüftpfannenprothese nach einem vorhergehenden Anspruch, **dadurch gekennzeichnet, dass** der Winkel ("α"), der zwischen der Kopplungsachse ("a") zwischen der äußeren Schale (12) und dem Einsatz (16, 116, 216) und der Orthogonale ("B") an die Bezugsebene (R) ausgebildet ist, als ein absoluter Wert dem Winkel zwischen der Achse der inneren Positionierauflagefläche (16) des Einsatzes und der Kopplungsachse ("a") gleich ist, so dass es zumindest eine Position des Einsatzes (16, 116, 216) gibt, die einen Abdeckwinkel von Null mit Bezug auf die Bezugsebene (R) definiert.

10. Hüftpfannenprothese nach einem der vorhergehenden Ansprüche, **dadurch gekennzeichnet, dass** das Ausrichtmittel ein Paar Vorsprünge oder Stifte (32, 132) umfasst, die auf der äußeren Oberfläche des Einsatzes (16, 116, 216) ausgebildet sind und mit ineinandergreifenden Rückhaltemitteln (33, 133) zusammenwirken können, die auf der inneren Oberfläche der äußeren Schale (12) vorhanden sind, um die vorbestimmten Positionen des Einsatzes (16, 116, 216) mit Bezug auf die Schale (12) zu definieren.

11. Hüftprothese nach einem der vorhergehenden Ansprüche, **dadurch gekennzeichnet, dass** der vorbestimmte Winkel ("α") einen Wert zwischen 5° und 45° hat.

12. Hüftpfannenprothese nach einem vorhergehenden Anspruch, **dadurch gekennzeichnet, dass** die Einsätze (116, 216) des zweiten und dritten Typs einen Anteverionswinkel von ungefähr 15° definieren können.

## Revendications

1. Prothèse acétabulaire de la hanche comprenant au moins une coque externe (12), pouvant être insérée et fixée dans un siège osseux correspondant, et un insert pouvant être inséré dans ladite coque externe (12) et recevoir un élément articulaire pouvant à son tour recevoir la tête d'une prothèse fémorale, **caractérisée en ce que** ledit insert (16, 116, 216) comprend des moyens d'alignement (32, 132) permettant de coupler de manière univoque ledit insert (16, 116, 216) à ladite coque externe (12) selon au moins deux positions alternatives prédéterminées, différant par leur orientation angulaire par rapport à au moins un plan de référence, lesdites au moins deux positions prédéterminées alternatives comprenant une première et une seconde position, seconde position dans laquelle ledit insert (16, 116, 216) pivote par rapport à ladite première position par rapport à un axe ("a") d'accouplement avec ladite coque externe (12), chacune desdites positions prédéfinies définissant une valeur respective et différente d'angle de recouvrement de l'insert (16, 116, 216) par rapport audit plan de référence.

2. Prothèse acétabulaire selon la revendication 1, **caractérisée en ce que** dans ladite seconde position, ledit insert (16, 116, 216) pivote par rapport à la première position de 180° par rapport à un axe ("a") d'accouplement avec ladite coque externe (12).

3. Prothèse acétabulaire selon la revendication 1 ou 2, **caractérisée en ce que** ledit insert (16, 11-, 216) a un siège de positionnement interne (16a) formant un angle prédéterminé (α), par rapport à l'axe d'accouplement ("a"), entre l'insert et la coque externe (12).

4. Prothèse acétabulaire selon la revendication 3, comprenant des moyens (20, 120) de fixation au siège osseux définissant un plan de référence (R) par rapport auquel est mesuré un angle de recouvrement dudit insert (16, 116, 216), **caractérisée en ce que** ledit axe d'accouplement ("a") entre la coque externe (12) et l'insert (16, 116, 216) est incliné dudit angle prédéterminé ("α") par rapport à la perpendiculaire audit plan de référence (R).

5. Prothèse acétabulaire selon l'une quelconque des revendications précédentes, **caractérisée en que** l'accouplement entre la coque externe (12) et l'insert (16, 116, 216) est de type conique avec un axe d'accouplement ("a").

6. Prothèse acétabulaire selon la revendication 4, **caractérisée en ce que** ledit insert est au moins d'un premier type (16) symétrique sur un plan frontal (Y) sensiblement vertical par rapport à la hanche, afin de faire varier l'angle de recouvrement par rapport audit plan de référence (R), dans le cas d'un angle d'antéversion de zéro sur un plan transversal (X) sensiblement horizontal par rapport à la hanche.

7. Prothèse acétabulaire selon la revendication 4, **caractérisée en ce que** ledit insert est respectivement d'un deuxième (116) et d'un troisième (216) type asymétrique par rapport au plan frontal (Y) sensiblement vertical par rapport à la hanche, afin de faire varier l'angle de recouvrement par rapport audit plan de référence (R), dans le cas d'un angle d'antéversion différent de zéro sur un plan transversal (X) sensiblement horizontal par rapport à la hanche.

8. Prothèse acétabulaire selon la revendication 7, **caractérisée en ce que** ledit insert (116) du deuxième type présente une conformation spéculaire par rapport audit insert (216) du troisième type, afin de définir des conditions de recouvrement spéculaire entre la hanche droite et la hanche gauche dans le cas d'un angle d'antéversion différent de zéro.

9. Prothèse acétabulaire selon l'une quelconque des revendications précédentes, **caractérisée en ce que** l'angle ("α") formé entre l'axe d'accouplement ("a") entre ladite coque externe (12) et ledit insert (16, 116, 216), et ladite perpendiculaire (b") au plan de référence (R) est égal, en valeur absolue, à l'angle formé entre l'axe du siège de positionnement interne (16a) dudit insert et ledit axe d'accouplement ("a"), de sorte qu'il existe au moins une position dudit insert (16, 116, 216) qui définisse un angle de recouvrement de zéro par rapport audit plan de référence (R).

10. Prothèse acétabulaire selon l'une quelconque des revendications précédentes, **caractérisée en ce que** lesdits moyens d'alignement comprennent deux saillies ou ergots (32, 132) réalisés sur la surface externe dudit insert (16, 116, 216) et pouvant coopérer avec des moyens de rétention associés (33, 133), présents sur la surface interne de ladite coque externe (12), afin de définir lesdites positions prédéterminées dudit insert (16, 116, 216) par rapport à ladite coque (12).

11. Prothèse acétabulaire selon l'une quelconque des revendications précédentes, **caractérisée en ce que** la valeur dudit angle prédéterminé (α) est comprise entre 5 et 45°.

12. Prothèse acétabulaire selon l'une quelconque des revendications précédentes, **caractérisée en ce que** lesdits inserts (116, 216) du deuxième et troisième type sont capables de définir un angle d'antéversion d'environ 15°.
